# EUROPEAN PATENT APPLICATION

(11) **EP 2 179 735 A1**
(43) Date of publication of application: **28.04.2010**
(21) Application number: 08778983.0
(22) Date of filing: 16.07.2008
(51) Int. Cl.: A61K 31/53, A61K 31/5513, A61P 25/08

(54) **PHARMACEUTICAL COMPOSITION COMBINING LAMOTRIGINE AND CLONAZEPAM, AND USE THEREOF FOR TREATING CONVULSIVE DISORDERS AND EPILEPTIC SYNDROMES**

(30) Priority: 16.07.2007 MX MX07008644
(71) Applicant: WORLD-TRADE IMPORT-EXPORT, WTIE, A.G., 6320 Zug (CH)
(72) Inventor: GARCÍA ARMENTA, María, Elena, Jalisco (MX); SANTOS MURILLO, Josefina, Jalisco (MX); ÁLVAREZ OCHOA, Victor, Guillermo, C.P. 45222 Zapopan Jalisco (MX)
(74) Representative: Carvajal y Urquijo, Isabel
(86) International application number: PCT/MX2008/000093
(87) International publication number: WO 2009/011561

(57) **Abstract**

The invention relates to a pharmaceutical composition consisting of the synergistic combination of an anticonvulsant agent such as the active ingredient Lamotrigine and a benzodiazepine agent such as the active ingredient clonazepam, said active ingredients being formulated in a single dosage unit to be orally administered in the form of capsules or tablets. Said composition can be used for the control and treatment of illnesses such as convulsive disorders, epileptic syndromes and other related illnesses.

## Description

### FIELD OF INVENTION

The present invention is applicable to pharmaceutical industry and describes a pharmaceutical composition comprising a synergistic combination of an anticonvulsant agent, such as the active principle: Lamotrigine and a benzodiazepine agent, such as active principle: Clonazepam, which are formulated in a single dosage unit, the same which is indicated for treatment and control of convulsive disorders and epileptic syndromes.

The combination of above mentioned active principles produces a larger synergistic effect when jointly administered in a single dose unit unlike when these are independently administered, causing benefits such as: lower concentrations of formulated active principles, lower administered doses, faster action, higher therapeutic effect efficacy and lower side effect risks.

### BACKGROUND OF INVENTION

Epilepsy affects about 100 to 200 million people worldwide. It constitutes a heterogeneous group of diseases and syndromes including an important group such as brain injury symptom; in a second group genetic causes play an important role in pathogenesis and thirdly, causes have not been determined (cryptogenic).

It is estimated that about 1 to 3% of population will have epilepsy at any time along life. According to a number of studies performed on population, epilepsy prevalence rate varies from 5 to 8 individuals per 1,000 inhabitants in industrialized countries, while 5 to 57 individuals per 1,000 inhabitants are present in developing countries, being the tenth cause of medical consultation in some of these countries.

Epilepsy is a chronic disease caused by brain gray substance which is manifested by the presence of a set of chronic neurological disorders having in common the existence of sudden and transient events of abnormal and synchronous discharges at any point in central nervous system with or without a consciousness loss; these events are called attack or fits and they may be of motor (convulsions or myoclonus), sensitive, autonomous or psychic (illusions or hallucinations) origin.

This disease is characterized by a sudden paroxystic depolarization with deviated movements of Na+, Ca++, K+ y Cl- in an unstable neuron population. Epileptic activity is caused by an excessive neuronal hypersynchrony, caused by biophysical cell alterations or by an abnormal synaptic transmission. Both mechanisms are involved in epileptogen focus genesis. The best epileptic event marker is paroxysmal depolarization deviation (DPS). When produced, neuron rest potential is raised above the normal action potential threshold. The first DPS period starts with Na+ channel opening and then depolarization, some second hundredths later an opening of Ca++ channels is produced, calcium enters into cytosol, attached to Ca++ fixing proteins and scavenged in mitochondria thus preventing an excessive calcium concentration which would be cytotoxic. Then, K+ channels are opened which produce the necessary repolarization for a new depolarization.

Antiepileptic drugs inhibit this sequence of events through a number of action mechanisms: a) Extension of inactivation period of voltage-dependent Na+ channels; b) Increase of gabaergic inhibition,- c) Calcium T channel blockage; d) Decrease of excitatory neurotransmitter action.

Epilepsy is represented by relapsing epileptic attacks (two or more) not caused by any immediate identified cause. Incidence of this condition is maximal in early ages, being stabilized in adult age and then increasing in the last decades of life. Common feature of all fits or seizures are the abnormal, excessive and hypersynchronous neuronal discharge in brain. Any epileptic attack has 2 elements, by one hand reticular excitation and its diffusion to the remaining brain cortex and on the other hand, the inhibitor system which finally suppresses it. Clinical epilepsy manifestations consist of sudden and transient abnormal phenomena which may include consciousness, motor, sensorial, autonomous or psychic event alterations, perceived by patients or by any observer. Some epidemiologic studies performed in several regions have detected epileptic attack histories in 3 to 10 per 1,000 inhabitants.

Active epilepsy is defined when a person with epilepsy has had at least one epileptic attack in the former 5 years independently of the used antiepileptic treatment. One case under treatment is that wherein a right epilepsy diagnosis has been made and which is receiving or has received an antiepileptic treatment up to date.

Multiple epileptic attacks (one or more) occurred in a term of 24 hours are considered as a single or isolated attack and though not considered as epilepsy by not being relapsing, they represent frequent problems and their cause shall be always investigated, since it might be the first attack of an epilepsy.

An epileptic status event has been defined as a single epileptic attack with a term longer than 30 minutes or a series of epileptic attacks with a term longer than 30 minutes wherein consciousness is not recovered between attacks. Epileptic states may be of convulsive (tonic-clonic) and non-convulsive (partial complex with absences).

Not all the sudden events compromising consciousness are of epileptic origin; a single convulsive attack and accidental convulsive attacks such as: fever convulsions, puerperal echlampsia, sob spasms, hysteria attack or syncopes, may be confused with epilepsy.

When currently talking about epileptic syndrome, this allows to classify a patient according to commencement age, attack features, critical and intercritical electroencephalographic tracing, permanent or non-permanent existence of neurological and psychic disorders and eventual etiology; thus allowing administering a right and suitable treatment, with future possibilities of long-term medication suppression.

Fever convulsions are present from 1 month up to 5 years old and they are associated with fever caused by an acute disease which causes these fever states, but without evidence that these are caused by intracranial infections or acknowledged neurological disease. Most of fever convulsions are short-term (less than 15 minutes) tonic-clonic or tonic type, and they are divided in single and complex. 80% are single type and they are characterized by: a) a period of less than 15 minutes, b) tonic-clonic or tonic generalized attacks, c) one single attack in the same fever period, d) no effects are left. Complex fever convulsion is characterized by: a) lasting more than 15 minutes, b) partial attacks, c) repeated in the same fever event, d) it may leave a neurological damage. Most children have a single fever convulsion along their life, but between 30 and 40% have a second one and just 10% have 3 or more attacks. Relapsing is more frequent among children who showed their first attack before the first year old without having demonstrated that attack relapsing increase the possibility of manifesting later epilepsy. Prophylactic treatment of fever convulsions is recommended in children who show one or more risk factors (familial epilepsy background, abnormal neurological development, complex fever convulsion).

During the recent years there has been a significant advancement in several areas related with epilepsy. On one hand, most of basic mechanisms for epilepsy attack are already known therefore, new pharmacological alternatives may be developed directed to stop the epileptic effects. There is currently a surprising change regarding to epilepsy treatment.

The need of introducing an antiepileptic treatment shall be individualized as a function of the risk factors shown by the subject when showing relapsing attacks. It is known that the risk factors for an epileptic attack relapse are: the presence of a brain injury, the existence of an EEG pattern with focal anomalies and concomitant mental retard; being these patients those who will show a higher risk of relapsing than the remaining normal population.

After orienting the research related with epilepsy preferably towards neurotransmitters, it has been known more about ionic channels during the last years, upon having identified aminoacid mutations which are part of the proteins comprised in ionic channels forming the substrate of a number of epilepsies.

Ionic channels are a heterogeneous class of proteinaceous complexes, responsible of generating and mediating signals among excitable cellular membranes. They are named in function of ion permeability and selectivity (Na⁺, Cl-, Ca⁺⁺, K⁺ channels) and respond to changes in membrane potential, to extracellular ligands or to second messengers.

### SUMMARY OF INVENTION

Ionic channels have a fundamental role in epilepsy. The channelopathies, that is, mutations in ionic channel structure and function may be a cause or substrate, both for idiopathic and acquired epilepsies, though 3 epileptic syndromes have been only currently confirmed: benign familial neonatal convulsions, generalized epilepsy with fever convulsions, dominant autosomal nocturnal front epilepsy. Incidence of these syndromes is very scarce though probably they have not been identified in many cases due to a lack of knowledge about their existence.

On the other hand, ionic channels have a relevant role in discharge synchronization and propagation produced by attacks, independently of the causes within them.

### Types of ionic channels:

1. Voltage-dependant ionic channels, responding to changes in membrane potential:
   - Voltage-dependant Ca++ channel.
   - Voltage-dependant Na+ channel.
   - Voltage-dependant K+ channel.
2. Ionic channels related with extracellular ligands:
   - With cholinergic nicotinic receptor: Na+ channel.
   - With GABAergic receptors: Cl- channel from GABA-A receptor.
   - With glutaminergic receptors: Na+ channel from AMPA receptor, Na+ channel from KA receptor, Ca++ channel from NMDA receptor.
3. Ionic channels linked to second messengers:
   - Ca++ channel linked to inositol triphosphate.
   - K+ channel of GABA-B receptor linked to protein G.

Knowledge of this information provides us a possibility to develop combined drugs, based on the knowledge of factors conditioning each epileptic syndrome and action mechanisms of anti-epileptic compounds.

In order to provide a pharmaceutical alternative to achieve a better life quality in patients who suffer diseases such as: convulsive disorders and epileptic syndromes, the development of a pharmaceutical composition below described was carried out.

### DETAILED DESCRIPTION OF INVENTION

Currently, most of drugs in marketplace for treatment of convulsive disorders and epileptic syndromes comprise active principles which are independently formulated, those which fulfill with a specific therapeutic activity; however, a significant part of patients demand at least two drugs to achieve an effective control in convulsive attacks; in this case, the activity of each of the active principles, their action mechanisms, possible interactions among them, synergistic activity shall be assessed, in order to be able to assess the possibility of reducing the frequency of the administered doses and to decrease the manifestation of side effects, especially because these treatments are long-term administered.

In the light of the above and in order to suppress all the drawbacks presented when administering independently the active principles, the development of a pharmaceutical composition subject of present invention was carried out, which comprises a synergistic combination of an anticonvulsant agent and a benzodiazepine agent,- which produces a satisfactory therapeutic effect when orally administered together in a single dosage unit unlike when they are independently administered, causing benefits such as: lower concentrations in formulated active principles, lower administered dosages, faster action, higher therapeutic effect efficacy and lower risk to show side effects.

Anti-convulsant agents or anti-epilepsy compounds (AE) are characterized by influencing the brain hyperexcitabillity, that is, its capacity to decrease the excitability of the excessively active nervous tissue. AE are proved efficacy drugs in epilepsy treatment. The term "antiepileptic" is used as a synonymous of anticonvulsant, though as being well known, not all epilepsies are convulsive.

These drugs may inhibit or decrease the appearance of convulsive attacks by 3 general mechanisms:
a) Increasing transmission by D-amino butyric acid intervention (GABA). b) Inhibiting neuronal exicitability, by inhibiting voltage-dependant sodium channels (Na+), c) Impeding voltage-dependant (Ca++) calcium channel activation, known as: calcium T channels.

Although most of AE which are used nowadays were discovered through their efficacy in animal models, there is no doubt that new knowledge about neurochemical phenomena involved in epilepsy shall favor drug development for its treatment. Drugs are known which also act by blocking the glutamate receptor (excitatory neurotransmitter) being under preclinical study phase.

Anti-epilepsy compounds increase GABA action by easing activation of GABA-A receptors (benzodiazepines and phenobarbital); by inhibiting GABA-transaminase, an enzyme in charge of GABA inactivation, they then increase their concentrations up to a synapse level (vigabatrine); by inhibiting GABA reuptake (tiagabine) and upon releasing GABA from presynaptic terminal (gabapentine).

GABA is the main inhibiting neurotransmitter found in brain, being synthesized therein from glutamate by action of glutamic acid decarboxilase enzyme, and being degraded by a transaminase. It has 2 receptor types: GABA-A y GABA-B. Stimulus from first one is linked to an increase in cellular membrane permeability to chloride ions and therefore, to a decrease in neuronal excitability. An increase in GABA mediated transmission, through any route, produces hyperpolarization and neuronal discharge decrease.

Anti-epilepsy compounds inhibit the function of voltage-dependant sodium channels (Na+): carbamazepine (CBZ), diphenylhydantoine (DFH), valproate and lamotrigine, are drugs frequently used which act by decreasing neuronal membrane excitability and generation of action potential (PA). This action is performed by blocking voltage-dependant sodium channels, responsible of PA generation. Blockage preferably occurs over cells which are repeatedly discharging, which happens in epileptic patients. The more discharge frequency in neurons, the higher is blockage (use-dependence phenomenon). Preference seems to be related with the capacity of these drugs to discriminate between different states from sodium channels (rest, inactive and open). AE drugs have preference by an inactive state of sodium channels and hinder their passage to rest state, a form which is available for depolarization.

Anti-epilepsy compounds hinder voltage-dependant (Ca++) calcium channels activation: etosuximide, a drug which single clinical prescription is for treatment of absence attacks, acts by preventing calcium entering into cells. This blocks voltage-dependant calcium channels (T channels), which prevents discharges from specific neuron groups. Part of AE action from valproate is also related with this mechanism, since small concentrations of this drug are able to inhibit these channels and justify their use as an alternative therapy in treatment of absence attacks.

An ideal AE which suppresses all epileptic seizures without causing any side effects (RA) is not available. Medicaments which are used nowadays are not capable of suppressing seizures in 20 to 30 % from patients, and quite frequently they cause RA, in the range from mild disturbances at CNS level, up to death by medular aplasia or liver damage. Monotherapy is not always possible, especially in patients where more than one type of seizures is present where the RA risk by appearance of drug interactions is considerably increased on them.

We call classic anti-epilepsy compounds to those drugs which marked the beginning of pharmacological treatment of epilepsy and which by resisting the time proof and having been used in millions of persons, they continue being the first choice in convulsive attack management. The following drugs are found among them: phenobarbital, phenytoin, Carbamazepine, Valproic acid, Benzodiazepines. Some features of this type of anti-epilepsy compounds are: high percentages of binding to plasma proteins; they are metabolized through an oxidative process producing active metabolites; manifesting complex pharmacokinetics features; having a narrow therapeutic range; showing adverse events depending on administered concentrations; existing a possibility to monitor its therapeutic action.

New anti-epilepsy compounds correspond to a recent drug generation which has been introduced into market in the last decades in order to essentially provide a treatment option for refractory epilepsies by having lower side effects, both from physical or cognitive type. The following drugs are among them: Lamotrigine, Gabapentine, Felbamate, Levetiracetam, Oxcarbazepine, Tiagabine, Topiramate, Vigabatrine and Pregabaline. Some features of this type of anti-epilepsy compounds are: lower percentage of attachment to plasma proteins; its metabolism is carried out through a non-oxidative process; complex pharmacokinetic features are manifested; they have an undefined therapeutic range; difficulty in monitoring its therapeutic action.

Lamotrigine is a broad-activity anticonvulsant or antiepileptic drug which further acts as antimaniac and anticyclic. It is used in a number of psychiatric diseases, especially epilepsy and bipolar disorder, being very effective for single and complex partial epileptic convulsion treatment and secondarily, in therapy-resistant generalized tonic-clonic convulsions with multiple drugs.

Lamotrigine exerts its action by blocking the voltage-dependant sodium channels, causing with this a blockage in sustained neuronal repetitive discharge and further inhibiting the release of excitatory neurotransmitters such as glutamate. It has special effect over voltage-dependant Na+ channels and on the other hand having activity over NMDA receptors controlling glutamate release and favoring gene expression and phosphorylation of a number of receptors and ionic channels. It may modulate neurons at central level preventing calcium influx, both through a loss of NMDA receptor blockage by magnesium, as well as over voltage-dependant calcium channels. Even if depolarization is produced, it may restore membrane potential opening potassium channels.

Lamotrigine is rapidly and fully absorbed by intestine. Upon being orally administered, its bioavailability is about 98%. It reaches a maximum plasma concentration from 1 to 4 hours after oral delivery. Lamotrigine administration combined with food delays maximum concentration time in blood; however, this does not reduce the total absorbed drug amount. Its attachment to plasma proteins is about 55%; however, higher drug toxicity has not been observed in hypoproteinemia states. Lamotrigine mean life is from 25 to 30 hours when singly administered.

Lamotrigine metabolism is hepatic. Its biotransformation mainly consists of a conjugation with hepatic glucuronidation, which is performed through UDP-glucoronyl-transferases, transformed into a conjugated N-glucuronide which is largely removed by renal route (urine), only 2% is removed without changes by feces. 10% from drug is unalteredly removed. Lamotrigine induces its own metabolism depending on dosage, and it has been demonstrated that this does not affect (induces or inhibits) pharmacokinetics or other epilepsy compound drug metabolism, nor that from metabolized by cytochrome P450 enzymes in liver. Its mean life is shorter in children than in adults, a significant data for dosage in these patients.

Most common adverse events when administering Lamotrigine are: diplopy (14 %), vertigo (14 %), somnolence (13 %), headache (12 %), ataxia (11 %), asthenia (10 %), skin rash (3%) and generalized allergic rash (2 to 3 %).

Benzodiazepines (BZD) are a drug class with anxiolytic, hypnotic, anticonvulsant, sedative, amnesic and myorelaxing (muscle relaxing) effects.

BZD are nervous system suppressing agents more selective than other drugs such as barbiturics. They particularly act over limbic, thalamic and hypothalamic system from central nervous system producing suppression in activity. BZDs share similar chemical structure and they have great affinity with the benzodiazepine receptor complex found in central nervous system. BZDs structurally show a six-element benzene ring attached to another seven-element diazepine ring. Each specific BZD will emerge by radical substitution in different positions.

Specific receptors found in CNS whereby BZDs exert their effect are part of the benzodiazepine-GABA receptor complex. D-aminobutyric acid (GABA) is a neurotransmitter with inhibiting action and its receptors are part of a inhibitory bidirectional system connected among several CNS areas. BZDs enhance inhibiting action mediated by GABA. GABA acts over several receptor subtypes called: GABA-A and GABA-B. GABA-A is a primary receptor subtype found in CNS whereby anxiolytics and sedatives act. Some benzodiazepine receptor subtypes seem to be coupled with GABA-A receptors. Three benzodiazepine receptor subtypes are known: those located in cerebellum and brain cortex named BNZl, those located in brain cortex and spinal cord named BNZ2 and those located in peripheral tissues (such as adrenal glands, kidneys, pineal gland and platelets) named BNZ3. Activation of BNZl induces sleep, while drugs which are attached to BNZ2 cause muscle relaxation, anticonvulsant activity and they affect memory. Benzodiazepines are nonspecifically attached to BNZl and BNZ2 receptors enhancing GABA effects.

Unlike barbiturics which increase GABA responses by keeping open for a longer time the chlorine channel (Cl-), benzodiazepines increase GABA effects which allows to keep open the chlorine channel in hyperpolarized cells and prevents a later excitation thereof.

BZD may be differentiated according to their action time, which may be: short-action (between 2 and 10 hours) and long-action (from 12 to 100 hours) as to its effect.

In order to perform BZD administration, it is important to know whether patient suffers or has symptoms related with: liver disease, alcoholism, brain diseases, little salivation (in children), glaucoma, hyperactivity, renal or lung diseases, miastenia gravis, porphyrias, pregnancy or sleep apnea.

Convulsions, fever, tremors, muscle weakness, reflex loss, intense asthenia, involuntary movements, short breathing, mucose dryness (oral, conjunctive, nasal), erithematous skin, arterial hypotension, slow heart rate, mild mental alterations or even confusion and coma may be present along treatment. Benzodiazepines cause dependency therefore they shall be used by short time periods. Upon cancelling a benzodiazepine treatment, it takes about three weeks for the body to get unaccustomed.

Clonazepam is a benzodiazepine drug with suppressing action over central nervous system, having several pharmacologic properties such as: anxiolytic, anticonvulsant, hypnotic, sedative, myorelaxing and amnesic.

Clonazepam acts as a CNS depressant, causing all the depression levels, from a mild sedation up to hypnosis, depending on dosage. Clonazepam stimulates GABA receptors (D-aminobutyric acid) in the upstream activating reticular system. Given that GABA is an inhibitor, receptor stimulation increases inhibition and blocks cortical and limb excitation, after stimulating the reticular formation from brain stem.

Clonazepam, as other BZD, is mainly metabolized by hepatic microsomal enzymes, undergoing microsomal oxidation (phase I) and then glucuronoconjugation (phase II). Most of BZD shall be firstly oxidated (active metabolites, phase I) and then conjugated (inactive metabolites, phase ID. Metabolic transformation of Clonazepam is produced by an oxidative hydroxylation and 7-nitro group reduction, forming 7-amino or 7-acetylamino compounds, which may be conjugated to create new metabolites. The main metabolite is 7-amino-clonazepam, with limited anticonvulsant activity. Other four metabolites have been further identified but in a lower proportion.

Clonazepam is rapidly and fully absorbed in gastrointestinal tract after oral administration. Maximum plasma concentrations are registered about 1 to 4 hrs after drug delivery. Its oral bioavailability is 90%. Clonazepam removal is slow since the active metabolites may remain in blood several days or even weeks, with persistent effects. Clonazepam is of intermediate half life, fluctuating between 30 and 40 hours. Its attachment to plasma proteins is high (85%); being metabolized in liver and excreted by renal way.

In a term from 4 to 10 days from 50 to 70% of one Clonazepam oral dose is removed through urine and from 10 to 30% through feces, almost exclusively in the form of free or conjugated metabolites. Less than 0.5 % is recovered in urine in the form of unaltered Clonazepam.

Clonazepam is indicated for treatment of: epileptic states, myoclonic attack, epileptic type absences (refractory to succinimides or valproic acid); tonic-clonic convulsive attack (generally associated with another anticonvulsant); panic disorders.

More frequent adverse reactions by using Clonazepam are: sleepiness, drowsiness, difficulties in psychomotor coordination (ataxia) and motor function, cognitive function impairment and depression. With a high dose administration orexigenic effects may be produced (increase of appetite), dysarthria (difficulty for speech) and ataxia (difficulty in walking). The following may also be present: nervousness, behavior alterations, uncommon tiredness and weakness, equilibrium loss and anterograde amnesia (especially at high doses). Alcohol consumption and other CNS depressants shall be prevented during Clonazepam treatment.

Geriatric and weakened patients, children and patients with hepatic disorders, are more sensitive to Clonazepam effects over CNS. In elder people, it is important to assess the presences and intensity of potentially risky side symptoms such as: sleepiness, drowsiness, clumsiness and instability. Clonazepam goes through placenta, therefore its use shall be prevented during pregnancy, particularly along the first pregnancy quarter. Clonazepam is excreted through breast milk thus its prescription shall be assessed during nursing period since it may cause sedation in newborns and possibly difficulties in feeding and weight loss.

In the long-term treatment children may present possible side effects over physical or mental development, which may not be apparent until several years later.

Clonazepam administration shall be assessed when there is a presence of acute alcohol poisoning, drug-dependency background, closed angle glaucoma, renal or liver dysfunction, severe mental depression, hypoalbuminemia, miastenia gravis, psychosis, porphyria, severe chronic obstructive pulmonar disease.

The anticonvulsant agent used in the pharmaceutical composition subject of present invention is active principle: Lamotrigine, which is present in the formulation in a concentration range from 25.0 mg to 500.0 mg, being preferably used a concentration from about 25.0 mg to 100.0 mg, per dose unit.

Benzodiazepine agent used in the pharmaceutical composition subject of present invention is active principle: Clonazepam, which is present in the formulation in a concentration range from 0.5 mg to 20.0 mg, being preferably used a concentration of about 0.5 mg to 2.0 mg, per dose unit.

The pharmaceutical composition protected by present invention is formulated to be orally administered in a single dosage unit in capsule or tablet form, wherein the synergistic combination of active principles: Lamotrigine y Clonazepam is contained, as well as pharmaceutically acceptable excipients.

Said pharmaceutical composition has been developed in order to provide a pharmaceutical alternative for control and treatment of diseases such as: convulsive disorders and epileptic syndromes, which provides significant advantages such as: lower active principle concentrations contained in formulation, lower administered dosages, faster action, higher therapeutic effect efficacy, a satisfactory synergistic effect which is translated into an efficient control of convulsive symptoms and lower risks that adverse events are manifested. Findings suggest that a combination of Lamotrigine y Clonazepam in a single dosage unit, produces a satisfactory synergistic effect for control and treatment of convulsive and epileptic attack, unlike when said active principles are administered separately.

In order to assess the efficacy and tolerance of the pharmaceutical composition subject of present invention, as well as the synergistic effect of active principles Lamotrigine and Clonazepam combined in a single dosage unit, a comparative clinical study was carried out wherein above mentioned active principles were administered, as well as a combination thereof.

### COMPARATIVE CLINICAL STUDY OF SYNERGY OF COMBINATION OF LAMOTRIGINE/CLONAZEPAM VS LAMOTRIGINE AND CLONAZEPAM INDEPENDENTLY ADMINISTERED.

Combined therapy in epilepsy is a common therapeutic objective in patients with refractory convulsions and for those whom monotherapy is unsuitable.

From a pharmacologic point of view, each combination of two fully active medicaments always evokes pharmacodynamic, pharmacokinetic nature interactions or a mixture thereof.

It has been accepted that medicament combinations of anti-epilepsy compounds which exert supra-additivity (synergy) as to anticonvulsant activity and with minimum or without side effects in animals are recommended as favorable for clinical practice such as: combinations of Lamotrigine and Valproate, Lamotrigine and Topiramate and Gabapentine and Carbamazepine have proved that patients with untreatable convulsive attacks were free from convulsions for more than 1 year.

Similarly, isobolographic experiments in mice have revealed that combinations of Lamotrigine and Valproate, Lamotrigine and Topiramate and Gabapentine and Carbamazepine synergically act in Convulsion Test produced by Electroshocks (MES); isobolographic studies also revealed at the same time the antagonism of Lamotrigine and Carbamazepina combinations in MES test in mice.

Studies of combinations with anti-epilepsy compound medicaments in rodents are predictors of their anticonvulsant effects in clinical practice and are of remarkable relevance for preselecting favorable combinations of anti-epilepsy compounds.

Many methods are currently available for analyzing the effects of combinations of two or more medicaments. The most commonly used isobolographic analysis was proposed by Lowe (1953) and was adapted by Tallarida (1992), who applied this method for assessing pharmacologic interactions between coadministered medicaments in combinations with several fixed ratios. This method has been accepted as the "gold standard" to detect medicament interactions in preclinical studies.

Theoretically, isobolographic studies may distinguish the 5 most important types of interactions:
1. Pure additivity.
2. Supra - additivity (synergy).
3. Indifference.
4. Sub-additivity (relative antagonism).
5. Infra-additivity (absolute antagonism).

This modern focus provides information to researchers about the exact interaction types exerted by two medicaments mixed for several estimated effects.

The 3D analysis is required for a full description and a clear identification of a dependent relationship of administered doses of two medicaments. For epilepsy research studies, this interaction analysis method is remarkably important to allow a determination of existing relationships among epilepsy compound medicaments.

Subject of this study is to monitor the interaction between Lamotrigine and Clonazepam with the MES test using the 3D isobolographic analysis.

MES test in rodents is broadly accepted as an experimental model of tonic-clonic convulsions or certain partial convulsions with or without secondary generalization. Brain concentrations were also assessed with anti-epilepsy compounds to monitor whether effects result in a pharmacodynamic and/or pharmacokinetic interaction.

### Materials and Methods.

Tests were carried out in Swiss, male, adult, albine mice with a weight between 22-26 gr. Lamotrigine and Clonazepam medicaments were suspended in 1% Tween 80 solutions and distilled water.

Firstly, separately administered medicament effects were studied. Lamotrigine effects were studied in 3 to 8 mg/kg doses and Clonazepam studied doses were 20, 25, 30, 35 and 40 mg/kg peritoneally administered, 60 minutes before MES test and 30 minutes before MES test, respectively (Table 1) Testing was later performed with a combination of Lamotrigine/ Clonazepam peritoneally administered 60 minutes before the MES test (Table 2).

### Results.

**Table 1. Anticonvulsant activity with Lamotrigine and Clonazepam drugs independently administered.**

| Drug | dose (mg/kg) | P/T | % |
|---|---|---|---|
| **LTG** | 3 | 0/8 | 0 |
| | 4 | 2/8 | 25 |
| | 5 | 4/8 | 50 |
| | 6 | 6/8 | 75 100 |
| | 8 | 8/8 | |
| | | | |
| **CZP** | 20 | 1/8 | 12.5 |
| | 25 | 2/8 | 25 |
| | 30 | 4/8 | 50 |
| | 35 | 6/8 | 75 |
| | 40 | 8/8 | 100 |
| Anticonvulsant activity of Lamotrigine (LTG) and Clonazepam (CZP) independently administered versus MES test; P= Number of protected animals against electroconvulsion; T= Total number of animals challenged with MES test; %= Percentage of protection against MES test. | | | |

**Table 2. Anticonvulsant activity of Lamotrigine / Clonazepam combination.**

| LTG | (mg/kg) % | | CZP (mg/kg) | Mixture (LTG + CZP) | P/T |
|---|---|---|---|---|---|
| 0.8 | | | 4.7 | 5.5 | 0/8 |
| | 0 | | | | |
| 1.0 | | | 5.7 | 6.7 | 2/8 |
| | | 25 | | | |
| 1.3 | | | 7.7 | 9.0 | 4/8 |
| | 50 | | | | |
| 2.3 | | | 13.8 | 16.1 | 6/8 |
| | | 75 | | | |
| 2.8 | | | 16.5 | 19.3 | 8/8 |
| | 100 | | | | |
| 1.4 | | | 2.8 | 4.2 | 0/8 |
| | | 0 | | | |
| 1.6 | | | 3.1 | 4.7 | 2/8 |
| | | 25 | | | |
| 2.1 | | | 4.1 | 6.2 | 4/8 |
| | | 50 | | | |
| 3.2 | | | 6.2 | 9.4 | 7/8 |
| | | 87 | | | |
| 3.6 | | | 7.1 | 10.7 | 8/8 |
| | | 100 | | | |

Effect of a combination of Lamotrigine / Clonazepam in induced mice convulsions (MES), mean efficient dosage. The mixture protects 50% of treated animals.

### Conclusions.

Results clearly indicate that Lamotrigine synergically interacts with Clonazepam as to its anticonvulsant activity against convulsions induced (MES) in mice.

Isobolographic analyses proved that the combinations showed supra-additive interactions.

## Claims

1. Pharmaceutical composition comprising a synergistic combination of an anticonvulsant agent and a benzodiazepine agent, **characterized in that** the anticonvulsant agent is active principle: Lamotrigine and the benzodiazepine agent is active principle: Clonazepam, as well as pharmaceutically acceptable excipients; wherein said active principles are present in the formulation in a concentration range from 25.0 mg to 500.0 mg for Lamotrigine and from 0.5 mg to 20.0 mg for Clonazepam; which are formulated in a single dosage unit to be orally administered, which is indicated for control and treatment of convulsive disorders, epileptic syndromes and related diseases.

2. Pharmaceutical composition according to claim 1, **characterized in that** the anticonvulsant agent, such as active principle: Lamotrigine is present in the formulation in a concentration range from 25.0 mg to 500.0 mg, being preferably used in the formulation in a concentration from 25.0 mg to 100.0 mg, per dose unit.

3. Pharmaceutical composition according to claims 1 and 2 **characterized in that** the benzodiazepine agent, such as active principle: Clonazepam is present in the formulation in a concentration range from 0.5 mg to 20.0 mg, being preferably used in the formulation in a concentration from 0.5 mg to 2.0 mg, per dose unit.

4. Pharmaceutical composition according to claims 1 to 3, **characterized in that** is formulated in a single dosage unit to be orally administered in capsule or tablet form.

5. The use of a pharmaceutical composition according to claims 1 a 4, **characterized in that** is indicated for control and treatment of diseases such as: convulsive disorders, epileptic syndromes and other related diseases.
